# EUROPEAN PATENT APPLICATION

(11) **EP 2 386 271 A1**
(43) Date of publication of application: **16.11.2011**
(21) Application number: 11164683.2
(22) Date of filing: 03.05.2011
(51) Int. Cl.: A61F 2/04, A61F 2/84

(54) **Plastic stent and stent operation apparatus for the same**

(30) Priority: 14.05.2010 KR 20100045343
(71) Applicant: Taewoong Medical Co., Ltd., Kyunggi do 415-873 (KR); Shin, Kyong-Min, Seoul (KR); Myung, Byung-Cheol, Kyongki-do (KR)
(72) Inventor: Shin, Kyong-Min, Seoul (KR); Myung, Byung-Cheol, Kyongki-do (KR)
(74) Representative: Wallin, Nicholas James

(57) **Abstract**

Disclosed herein are a plastic stent (10) and a stent operation apparatus (20) for plastic stents. The stent operation apparatus includes a stent push rod (21) having an externally threaded end (23). The stent body has an internal thread (26) on its circumferential inner surface. The plastic stent is coupled to the stent push rod by the threaded engagement between the externally threaded end and the internal thread, is inserted into the body of a patient, is adjusted in location by pushing or pulling the stent push rod, and is released from the stent push rod at a correct location in the body of the patient by disengaging the threaded engagement.

## Description

### BACKGROUND OF THE INVENTION

### 1. Field of the Invention

The present invention relates generally to plastic stents and stent operation apparatuses and, more particularly, to a plastic stent and a stent operation apparatus which is thread-coupled to the plastic stent, so that the plastic stent can be repeatedly extracted from and retracted into the stent operation apparatus, thus making it possible to accurately adjust a location at which the plastic stent is implanted into the body of a patient.

### 2. Description of the Related Art

Generally, a variety of plastic stents have been used to, for example, expand a contracted blood vessel and keep it in the expanded state, or expand various kinds of organ ducts, such as bile tracts, esophagi, respiratory tracts or ureters, which have become constricted, for example, by lesions occurring on the organ ducts or by temporary inflammations resulting from injuries caused when endoscopes are inserted into the organ ducts to treat lesions occurring in the bodies of patients.

To implant such a plastic stent into the body of a patient, the plastic stent is inserted into a stent operation apparatus before it is inserted into an organ of the patient's body. Here, the plastic stent must be extracted from and retracted into the stent operation apparatus so that the plastic stent can be implanted at the accurate location in the organ.

However in the conventional operation method in which the plastic stent can only be pushed out of the stent operation apparatus, after the plastic stent has been completely extracted from the stent operation apparatus one time, the location of the plastic stent cannot be readjusted because it cannot be retracted back into the stent operation apparatus.

Therefore, a plastic stent and a stent operation apparatus are required, which can be thread coupled to each other so that the plastic stent can be extracted into and retracted from the stent operation apparatus so that the position of the plastic stent can be accurately adjusted to its correct location in the body of a patient.

### SUMMARY OF THE INVENTION

Accordingly, the present invention has been made keeping in mind the above problems occurring in the prior art, and an object of the present invention is to provide a plastic stent and a stent operation apparatus which is thread-coupled to the plastic stent so that the location at which the plastic stent is implanted into the body of a patient can be accurately controlled, and the plastic stent can be easily implanted in the patient's body only by disengaging the threaded-coupling.

Another object of the present invention is to provide a plastic stent which can prevent a passage in the body from being clogged by infected bile so that the infected bile smoothly flows along the passage.

In order to accomplish the above object, in an aspect, the present invention provides a plastic stent, including a stent body with a flow hole formed through the stent body in a longitudinal direction thereof, the plastic stent being used by a stent operation apparatus, the stent operation apparatus comprising a stent push rod having an externally threaded end provided by forming an external thread on a circumferential outer surface of a distal end of the stent push rod. The stent body has an internal thread on a circumferential inner surface of the flow hole so that the internal thread engages with the externally threaded end of the stent push rod of the stent operation apparatus, wherein the plastic stent is coupled to the stent push rod by the threaded engagement between the externally threaded end of the stent push rod and the internal thread of the stent body, is inserted into a body of a patient, is adjusted in location by pushing or pulling the stent push rod, and is released from the stent push rod at a correct location in the body of the patient by disengaging the threaded engagement.

In another aspect, the present invention provides a stent operation apparatus for a plastic stent, the plastic stent comprising a stent body having a flow hole formed through the stent body in a longitudinal direction thereof, with an internal thread formed on a circumferential inner surface of the flow hole of the stent body. The stent operation apparatus includes a stent push rod having an externally threaded end provided by forming an external thread on a circumferential outer surface of a distal end of the stent push rod so that externally threaded end engages with the internal thread of the flow hole of the stent body, wherein the plastic stent is coupled to the stent push rod by the threaded engagement between the externally threaded end of the stent push rod and the internal thread of the stent body, is inserted into a body of a patient, is adjusted in location by pushing or pulling the stent push rod, and is released from the stent push rod at a correct location in the body of the patient by disengaging the threaded engagement.

### BRIEF DESCRIPTION OF THE DRAWINGS

The above and other objects, features and advantages of the present invention will be more clearly understood from the following detailed description taken in conjunction with the accompanying drawings, in which:
FIG. 1 is an exploded perspective view showing a plastic stent and a stent operation apparatus, according to an embodiment of the present invention;
FIG. 2 is a sectional view of the plastic stent according to the present invention;
FIG. 3 is a sectional view showing the coupling of the plastic stent to the stent operation apparatus according to the present invention;
FIG. 4 is a sectional view showing insertion of the plastic stent into an insert tube of the stent operation apparatus according to the present invention;
FIG. 5 is a view showing an example of insertion of the plastic stent of the present invention into a bile duct; and
FIG. 6 is a view showing discharge of infected bile through the plastic stent of the present invention that has been inserted into the bile duct.

### DESCRIPTION OF THE PREFERRED EMBODIMENTS

Hereinafter, a preferred embodiment of the present invention will be described in detail with reference to the attached drawings.

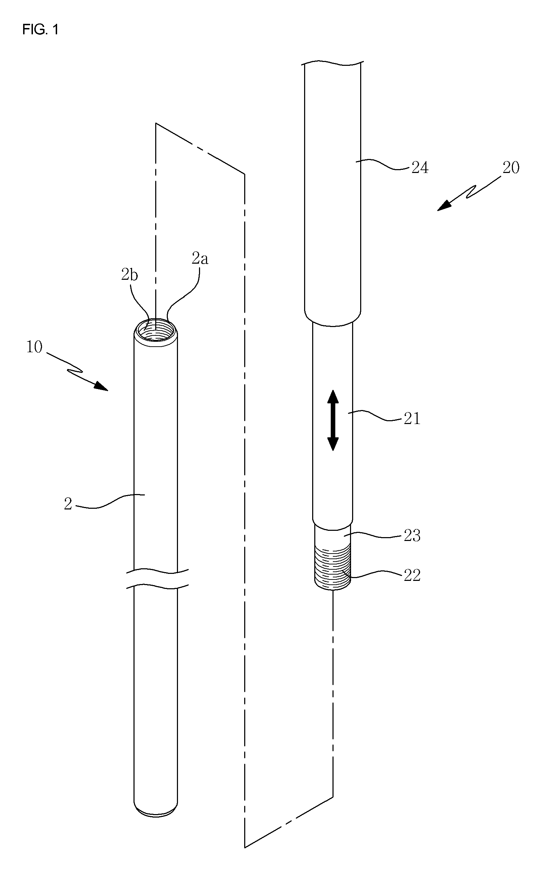

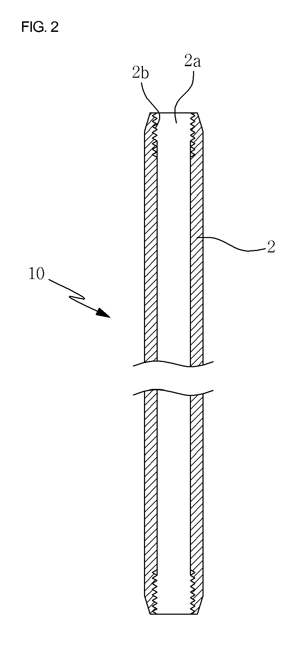

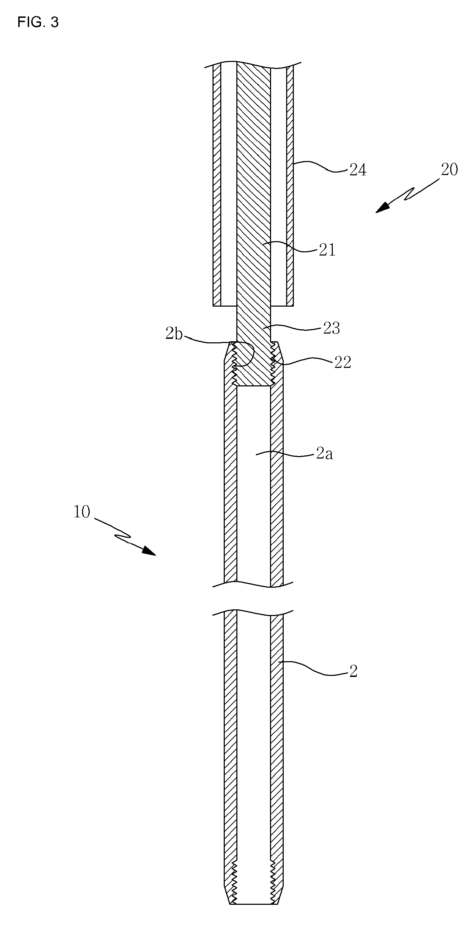

As shown in FIGS. 1 through 4, the present invention is related to a plastic stent 10 and a stent operation apparatus 20. The plastic stent 10 includes a stent body 2 which has a flow hole 2a that proceeds along a longitudinal axis of the stent body 2. The plastic stent 10 is inserted into a blood vessel or an organ of the body of a patient and used to discharge bile through the flow hole 2a of the stent body 2.

The plastic stent 10 is generally manufactured in a straight shape, a coil shape or a shape of a straight body with a hook.

In this embodiment, an internal thread 2b is formed on a circumferential inner surface of the flow hole 2a of the stent body 2.

Preferably, the internal thread 2b may be formed on the circumferential inner surface of one end of the flow hole 2a or on the circumferential inner surface of both ends thereof.

Furthermore, the stent operation apparatus 20, such as a catheter, includes a stent push rod 21 which has on a front end thereof an externally threaded end 23 on which an external thread 22 is formed.

The operation of inserting the plastic stent 10 proceeds in such a way that the plastic stent 10 is coupled to the stent operation apparatus 20 by engaging the internal thread 2b of the flow hole 2a with the external thread 23 of the stent push rod 21, is inserted into a vessel or an organ, is located at a correct position, and then is left in the vessel or the organ by disengaging the threaded coupling.

As such, the plastic stent 10 is coupled to the stent operation apparatus 20 by the threaded coupling between the external thread 23 and the internal thread 2b. Thus, when inserting the plastic stent 10 into the vessel or the organ, even after the plastic stent 10 has been completely extracted from the stent operation apparatus 20, it can be retracted again into the stent operation apparatus 20 to adjust the location thereof, unlike the conventional operation method in which only pushing the stent out of the stent operation apparatus can be performed. Therefore, the plastic stent 10 can be accurately located at the correct position.

The operation of inserting the plastic stent 10 of the present invention having the above-mentioned construction into a vessel of the body of a patient or a bile duct, and the effect of the operation will be explained.

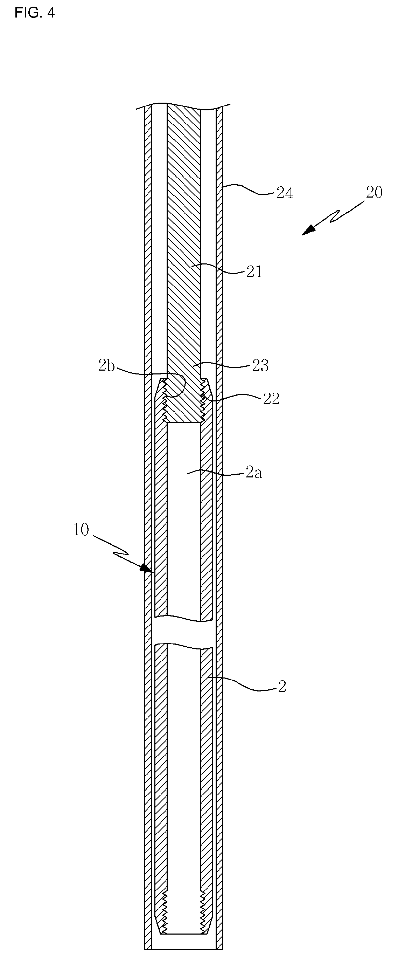

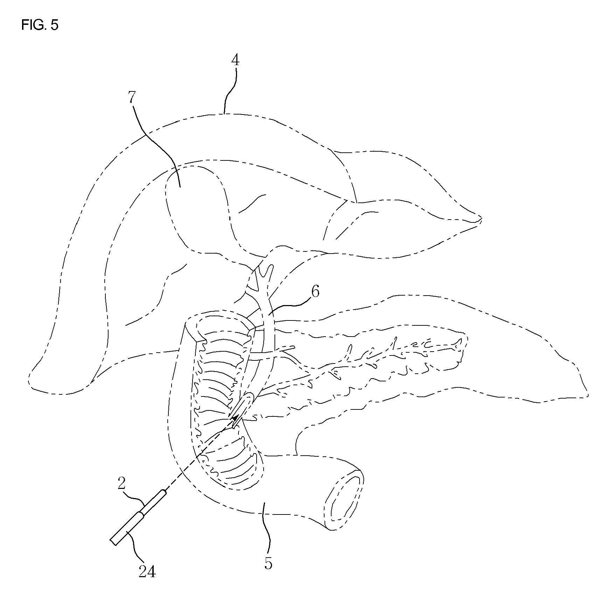

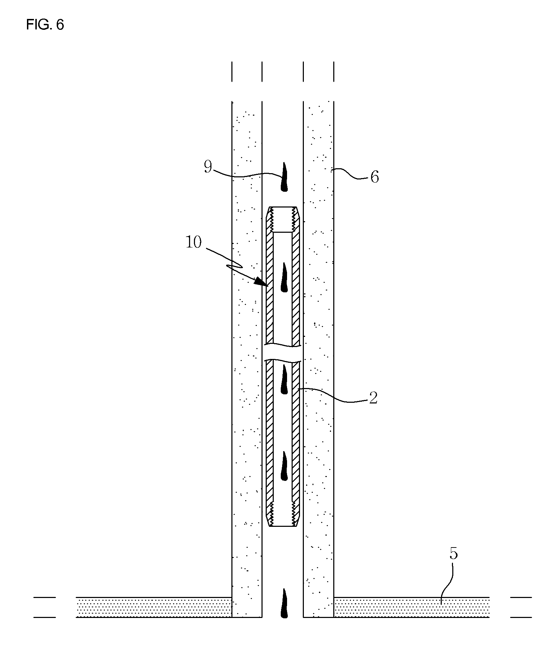

As shown in FIGS. 4 through 6, a bile duct 6 is a passage through which bile created in the gall bladder 7 of a liver 4 is supplied into the duodenum 5. An inflammation may occur in the bile duct 6 for a variety of reasons, such as physical damage, chemical damage attributable to treatment medicines, infection, hypersensitivity reaction, thermal damage, etc.

The inflammation in the bile duct 6 contaminates bile and disturbs the flow of bile from the bile duct 6 into the duodenum 5. In this case, the stent operation is performed.

A process of inserting the plastic stent 10 will be described. First the external thread 22 formed on the externally threaded end 23 of the stent push rod 21 of the stent operation apparatus 20 engages with the internal threaded end 2b of the flow hole 2a of the plastic stent 10.

Thereafter, the plastic stent 10 is inserted into an insert rod 24 of the stent operation apparatus 20 by pulling the stent push rod 21.

After preliminary steps preceding the stent insertion operation have been completed, the insert rod 24 into which the stent push rod 21 has been retracted is inserted into the body of the patient, for example, into the bile duct 6.

Subsequently, when the plastic stent 10 reaches a desired location, the stent push rod 21 of the stent operation apparatus 20 is pushed forwards so that the stent push rod 21 is extracted out of the insert rod 24.

Here, because the plastic stent 10 is thread-coupled to the stent push rod 21 by threaded-coupling, the location at which the plastic stent 10 is set in the body of the patient can be accurately adjusted by pushing the stent rod 21 out of the insert rod 24 or pulling it thereinto.

After the correct location of the stent rod 21 has been determined, the plastic stent 10 is completely extracted from the insert rod 24 by pushing the stent push rod 21. Then, the plastic stent 10 is brought into close contact with the inner surface of the bile duct 6 and reliably supported thereby.

After the location adjustment of the plastic stent 10 has been completed, the stent push rod 21 that has been thread-coupled to the plastic stent 10 is slowly rotated so that the plastic stent 10 is released from the stent push rod 21. Thereafter, the stent operation apparatus 20 is removed from the body of the patient, thus completing the stent insertion operation.

In this state, infected bile 9 which enters the bile duct 6 flows along the flow hole 2a of the plastic stent 10 before being discharged out of the body of the patient via the duodenum 5, the small intestine and the large intestine.

The status of infected bile 9 which is discharged from the patient's body for a predetermined duration is inspected. If infected bile no longer comes out, the stent operation apparatus is reinserted into the patient's body. Subsequently the stent push rod 21 is thread-coupled to the plastic stent 10 before the stent operation apparatus along with the plastic stent is removed from the patient's body.

As described above, a plastic stent of the present invention can prevent a passage in the body from being clogged with infected bile so that the infected bile smoothly flows along the passage.

Furthermore, the plastic stent is thread-coupled to a stent operation apparatus before a stent operation is performed. Therefore, the location at which the plastic stent is implanted into the body of a patient can be accurately controlled, and the plastic stent can be easily implanted into the patient's body only by disengaging the threaded-coupling.

Although the preferred embodiment of the present invention has been disclosed for illustrative purposes, those skilled in the art will appreciate that various modifications, additions and substitutions are possible, without departing from the scope and spirit of the invention as disclosed in the accompanying claims. For example, plastic material is not limited to a special material so long as it can attain the intended purpose and effect of the present invention. As necessary, metal which is harmless to the human body may be used.

## Claims

1. A plastic stent, comprising a stent body with a flow hole formed through the stent body in a longitudinal direction thereof, the plastic stent being used by a stent operation apparatus, the stent operation apparatus comprising a stent push rod having an externally threaded end provided by forming an external thread on a circumferential outer surface of a distal end of the stent push rod,
the stent body having an internal thread on a circumferential inner surface of the flow hole so that the internal thread engages with the externally threaded end of the stent push rod of the stent operation apparatus,
wherein the plastic stent is coupled to the stent push rod by the threaded engagement between the externally threaded end of the stent push rod and the internal thread of the stent body, is inserted into a body of a patient, is adjusted in location by pushing or pulling the stent push rod, and is released from the stent push rod at a correct location in the body of the patient by disengaging the threaded engagement.

2. The plastic stent as set forth in claim 1, wherein the internal thread is formed on one end of the flow hole.

3. The plastic stent as set forth in claim 1, wherein the internal thread comprises internal threads formed on respective opposite ends of the flow hole.

4. A stent operation apparatus for a plastic stent, the plastic stent comprising a stent body having a flow hole formed through the stent body in a longitudinal direction thereof, with an internal thread formed on a circumferential inner surface of the flow hole of the stent body,
the stent operation apparatus comprising a stent push rod having an externally threaded end provided by forming an external thread on a circumferential outer surface of a distal end of the stent push rod so that externally threaded end engages with the internal thread of the flow hole of the stent body,
wherein the plastic stent is coupled to the stent push rod by the threaded engagement between the externally threaded end of the stent push rod and the internal thread of the stent body, is inserted into a body of a patient, is adjusted in location by pushing or pulling the stent push rod, and is released from the stent push rod at a correct location in the body of the patient by disengaging the threaded engagement.
